(19) **Europäisches Patentamt European Patent Office Office européen des brevets**

(11) **EP 4 666 945 A1**

(12) **EUROPEAN PATENT APPLICATION**

(43) Date of publication:
24.12.2025 Bulletin 2025/52

(21) Application number: 25183808.2

(22) Date of filing: 18.06.2025

(51) International Patent Classification (IPC):
$A61B\ 5/16^{(2006.01)}$  $A61B\ 5/00^{(2006.01)}$
$G06N\ 3/049^{(2023.01)}$  $G16H\ 50/20^{(2018.01)}$
$G16H\ 50/50^{(2018.01)}$

(52) Cooperative Patent Classification (CPC):
A61B 5/7264; A61B 5/16; G06N 3/049;
G06N 3/088; G16H 50/20; G16H 50/50;
G16H 50/70

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH LA MA MD TN

(30) Priority: 20.06.2024 IN 202421047653

(71) Applicant: Tata Consultancy Services Limited
Mumbai, Maharashtra 400 021 (IN)

(72) Inventors:
• PARI, BHUVANAMBIGA
700135 Kolkata, West Bengal (IN)
• CHAKRAVARTY, KINGSHUK
700135 Kolkata, West Bengal (IN)
• SINHA, ANIRUDDHA
700135 Kolkata, West Bengal (IN)
• ROY, SANGHEETA
700135 Kolkata, West Bengal (IN)
• KUMAR, ARVIND
114 28 Stockholm (SE)

(74) Representative: Goddar, Heinz J.
Boehmert & Boehmert
Anwaltspartnerschaft mbB
Pettenkoferstrasse 22
80336 München (DE)

(54) **METHOD AND SYSTEM TO IDENTIFY NEURONAL ENSEMBLES IN BASAL GANGLIA USING HIERARCHICAL DRIFT-DIFFUSION MODELING**

(57) The present invention generally relates to the field of computational brain modeling. It is challenging to analyse neuronal activities that governs neuronal dynamics during the decision-making process using conventional techniques. Thus, embodiments of present disclosure provide a method and system to identify neuronal ensembles in basal ganglia using hierarchical drift-diffusion modeling. Microelectrode recording data of neuronal activity of neurons in the nuclei within BG of a subject are obtained. Then, spikes and associated spike times are extracted from the obtained data using which Inter-Spike Intervals (ISIs) for each of the neurons are calculated. Response times of each neuron is determined based on the ISIs and they are classified as one of an active state and a resting state which inherently reflected the broader network states responsible for behavioral responses by the neurons. Finally, the neurons are grouped into neuronal ensembles based on HDDM latent variables like drift rate.

200

Obtaining a microelectrode recording data of neuronal activity of a plurality of neurons in a plurality of nuclei within basal ganglia of a subject — 202

Pre-processing the microelectrode recording data to identify a plurality of spikes and associated spike times for each of the plurality of neurons — 204

Computing a plurality of Inter-Spike Intervals (ISIs) for each of the plurality of neurons based on the associated spike times — 206

Determining a response time of each of the plurality of neurons by adding the ISIs within a plurality of non-overlapping time windows from among the plurality of ISIs — 208

Classifying response of each of the plurality of neurons as one of: i) an active state, and ii) a resting state, based on a comparison of the determined response time of each of the plurality of neurons with a cutoff value, to obtain a classified state of each of the plurality of neurons — 210

Determining a plurality of parameters using hierarchical drift-diffusion modeling based on the determined response time and the classified state of each of the plurality of neurons — 212

Identifying a plurality of neuronal ensembles using a clustering technique based on the determined plurality of parameters — 214

FIG. 2

EP 4 666 945 A1

**Description**

CROSS-REFERENCE TO RELATED APPLICATIONS AND PRIORITY

The present application claims priority from Indian provisional patent application no. 202421047653, filed on June 20, 2024.

TECHNICAL FIELD

The present invention generally relates to the field of computational brain modeling, and, more particularly, to a method and system to identify neuronal ensembles in basal ganglia using hierarchical drift-diffusion modeling.

BACKGROUND

The Basal Ganglia (BG) is a crucial region of the brain, which is involved in decision making, evaluating options based on past experiences, selecting actions and executing them. Even seemingly random movements involve decision making through the integration of various signals by the BG to guide behavior towards a chosen course of action or movement. Parkinson's disease (PD) arises due to disruptions in the production of neuromodulator dopamine which causes alterations in neuronal properties and synaptic connections. Dopamine depletion-induced reorganization of BG circuits (BG networks) results in impairment of its ability to process information and make decisions in PD. Experimental studies have revealed the emergence of neuronal ensembles (alternately referred to as neuronal clusters) within Sub-Thalamic Nucleus (STN), Globus Pallidus externa (GPe), and Globus Pallidus interna (GPi) of the BG during information processing or generation of beta-band oscillations, a prominently observed feature in PD. Neurons within a nucleus, despite their diverse spatial origins and heterogeneous characteristics (such as firing patterns, neuronal properties, and synaptic connection profiles), can still cluster together based on the intrinsic dynamics governing the membrane potential. This heterogeneity is reflected in the spike trains of neurons in the BG network which is driven by local excitatory and inhibitory connections among neurons, as well as excitatory connections from outside the BG network. The spike train dynamics of individual neurons are significantly influenced by the variability of synaptic inputs received by the neuron. The synaptic current arriving at the soma of a neuron contains i) a drift part, due to the average firing rate of all neurons local and external to the network, and ii) a diffusion part, due to the stochastic arrival of spikes at the soma. Hence, the membrane potential evolution of the neuron can be described by a Fokker-Planck equation where drift is usually represented by a first-order differential operator, while diffusion is typically characterized by a second-order differential operator. Drift-diffusion-like signal activity has been observed in neurons belonging to the lateral interparietal cortex in prior studies of decision making in visual discrimination tasks. However, it is challenging to analyze neuronal activities that governs neuronal dynamics during the decision-making process since it requires some hidden variables which cannot be directly observed or recorded from neuronal firing patterns.

SUMMARY

Embodiments of the present disclosure present technological improvements as solutions to one or more of the above-mentioned technical problems recognized by the inventors in conventional systems. For example, in one embodiment, a method and system to identify neuronal ensembles in basal ganglia using hierarchical drift-diffusion modeling is provided. The method includes obtaining a microelectrode recording data of neuronal activity of a plurality of neurons in a plurality of nuclei within basal ganglia of a subject and pre-processing the microelectrode recording data to identify a plurality of spikes and associated spike times for each of the plurality of neurons. Further, the method includes computing a plurality of Inter-Spike Intervals (ISIs) for each of the plurality of neurons based on the associated spike times and determining a response time of each of the plurality of neurons by adding the ISIs within a plurality of non-overlapping time windows from among the plurality of ISIs. The method further includes classifying response of each of the plurality of neurons as one of: i) an active state, and ii) a resting state, based on a comparison of the determined response time of each of the plurality of neurons with a cutoff value, to obtain a classified state of each of the plurality of neurons. Further, the method includes determining a plurality of parameters using hierarchical drift-diffusion modeling based on the determined response time and the classified state of each of the plurality of neurons. Furthermore, the method includes identifying a plurality of neuronal ensembles using a clustering technique based on the determined plurality of parameters.

In another aspect, a system to identify neuronal ensembles in basal ganglia using hierarchical drift-diffusion modeling is provided. The system includes: a memory storing instructions; one or more communication interfaces; and one or more hardware processors coupled to the memory via the one or more communication interfaces, wherein the one or more hardware processors are configured by the instructions to: obtain a microelectrode recording data of neuronal activity of a plurality of neurons in a plurality of nuclei within basal ganglia of a subject and pre-process the microelectrode recording data to identify a plurality of spikes and associated spike times for each of the plurality of neurons. Further, the one or more hardware processors are configured by the instructions to compute a plurality of Inter-Spike Intervals (ISIs) for each of the plurality of neurons based on the associated spike times and determine a response time of each of the plurality of neurons by adding the ISIs within a plurality of non-overlapping time windows from among the plurality of ISIs. The one or more hardware processors are further configured by the instructions to classify response of each of the plurality of neurons as one of: i) an active state, and ii) a resting state, based on a comparison of the determined response time of each of the plurality of neurons with a cutoff value, to obtain a classified state of each of the plurality of neurons. Further, the one or more hardware processors are configured by the instructions to determine a plurality of parameters using hierarchical drift-diffusion modeling based on the determined response time and the classified state of each of the plurality of neurons. Furthermore, the one or more hardware processors are configured by the instructions to identify a plurality of neuronal ensembles using a clustering technique based on the determined plurality of parameters.

In yet another aspect, there are provided one or more non-transitory machine-readable information storage mediums comprising one or more instructions which when executed by one or more hardware processors cause a method to identify neuronal ensembles in basal ganglia using hierarchical drift-diffusion modeling. The method includes obtaining a microelectrode recording data of neuronal activity of a plurality of neurons in a plurality of nuclei within basal ganglia of a subject and pre-processing the microelectrode recording data to identify a plurality of spikes and associated spike times for each of the plurality of neurons. Further, the method includes computing a plurality of Inter-Spike Intervals (ISIs) for each of the plurality of neurons based on the associated spike times and determining a response time of each of the plurality of neurons by adding the ISIs within a plurality of non-overlapping time windows from among the plurality of ISIs. The method further includes classifying response of each of the plurality of neurons as one of: i) an active state, and ii) a resting state, based on a comparison of the determined response time of each of the plurality of neurons with a cutoff value, to obtain a classified state of each of the plurality of neurons. Further, the method includes determining a plurality of parameters using hierarchical drift-diffusion modeling based on the determined response time and the classified state of each of the plurality of neurons. Furthermore, the method includes identifying a plurality of neuronal ensembles using a clustering technique based on the determined plurality of parameters.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system to identify neuronal ensembles in basal ganglia using hierarchical drift-diffusion modeling, according to some embodiments of the present disclosure.

FIG. 2 is a flow diagram illustrating a method to identify neuronal ensembles in basal ganglia using hierarchical drift-diffusion modeling, according to some embodiments of the present disclosure.

FIGS. 3A and 3B, collectively referred to as FIG.3, illustrate spike times and response times of a plurality of neurons, respectively, according to some embodiments of the present disclosure.

FIG. 4A illustrates posterior probability distribution of drift rate for a neuron 'H0222' in Globus Pallidus externa (GPe) of the basal ganglia, obtained from Hierarchical Drift-Diffusion Modeling (HDDM), according to some embodiments of the present disclosure.

FIG. 4B illustrates Bhattacharyya distance calculated between drift rate posteriors of each pair of neurons, according to some embodiments of the present disclosure.

FIG. 4C illustrates neuronal ensembles with similar drift rate posterior distributions, according to some embodiments of the present disclosure.

FIG. 5A illustrates evolution of average silhouette score of a plurality of neurons in GPe in Parkinson's Disease (PD) condition with increase in radius of neuronal ensembles for spike count threshold $\theta_B$ = 30 spikes, according to some embodiments of the present disclosure.

FIG. 5B illustrates 3 neuronal ensembles (clusters) having least fraction of uncorrelated neurons among the neuronal ensembles illustrated in FIG. 5A, according to some embodiments of the present disclosure.

FIG. 6 illustrates a plurality of neuronal ensembles in GPe in healthy condition and their corresponding Silhouette scores, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The Basal Ganglia (BG) plays a pivotal role in movement-related decision-making. BG can be viewed as interconnected nuclei linked through synaptic connections, with each nucleus comprising connected neurons. This entire system of connected neurons is referred to as a BG network. Within this broader BG network, the STN-GPe loop represents a subnetwork. In Parkinson's disease (PD) like scenario, neuronal properties and network connectivity get altered. This leads to aberrant neuronal spiking characteristics affecting the overall oscillatory dynamics of the network. Classically, the rate of change (drift) in the membrane potential and the variation (diffusion) of the same across multiple spikes are modelled using drift-diffusion framework. During active state of a subject, the movement due to behavioural responses are a result of sustained spiking of multiple neurons within a nucleus. The diversity within a nucleus leads to formation of groups of neurons having similar dynamics. However, relation between the diversity in the neuronal responses and the movement behaviour are not well studied. It is challenging to analyse neuronal activities that governs neuronal dynamics during the decision-making process since it requires some hidden variables which cannot be directly observed or recorded from neuronal firing patterns.

In order to overcome the above-mentioned drawbacks of conventional techniques, embodiments of present disclosure provide a method and system to identify neuronal ensembles in Basal Ganglia (BG) using Hierarchical Drift-Diffusion Modeling (HDDM). Microelectrode recording (MER) data of neuronal activity of neurons in the nuclei within BG of a subject are obtained. Then, spikes and associated spike times are extracted from the obtained MER data using which Inter-Spike Intervals (ISIs) for each of the neurons are calculated. Response times of each neuron is determined based on the ISIs and they are classified as one of an active state and a resting state which inherently reflect the broader network states responsible for behavioral responses by the neurons. Finally, the neurons are grouped into neuronal ensembles based on HDDM latent variables (alternatively referred as parameters) like drift rate. The method of present disclosure was applied on publicly available ISI data and findings revealed discrete clusters (alternatively referred as neuronal ensembles) in regions like the Globus Pallidus externa (GPe), Globus Pallidus interna (GPi), and Subthalamic Nucleus (STN) in the BG. Results demonstrated well-formed clusters using the latent information of HDDM which were not revealed using directly observable parameters such as revised local variation and instantaneous firing rate.

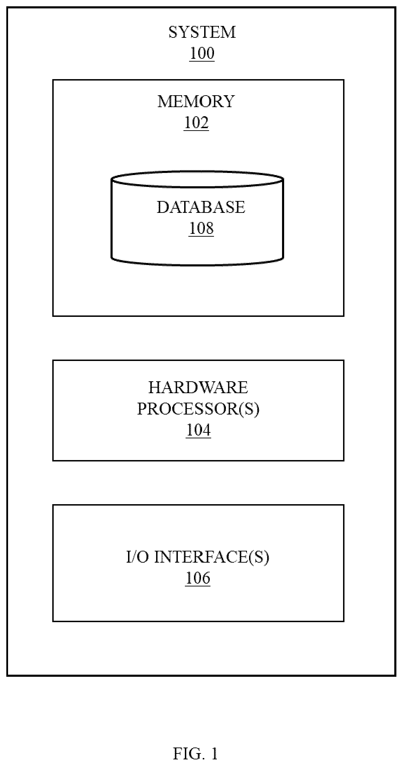

Referring now to the drawings, and more particularly to FIGS. 1 to 6, where similar reference characters denote corresponding features consistently throughout the figures, there are shown preferred embodiments, and these embodiments are described in the context of the following exemplary system and/or method.

FIG. 1 illustrates an exemplary block diagram of a system to identify neuronal ensembles in basal ganglia using hierarchical drift-diffusion modeling, according to some embodiments of the present disclosure. In an embodiment, the system 100 includes one or more processors 104, communication interface device(s) 106 or Input/Output (I/O) interface(s) 106 or user interface 106, and one or more data storage devices or memory 102 operatively coupled to the one or more processors 104. The one or more processors 104 that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

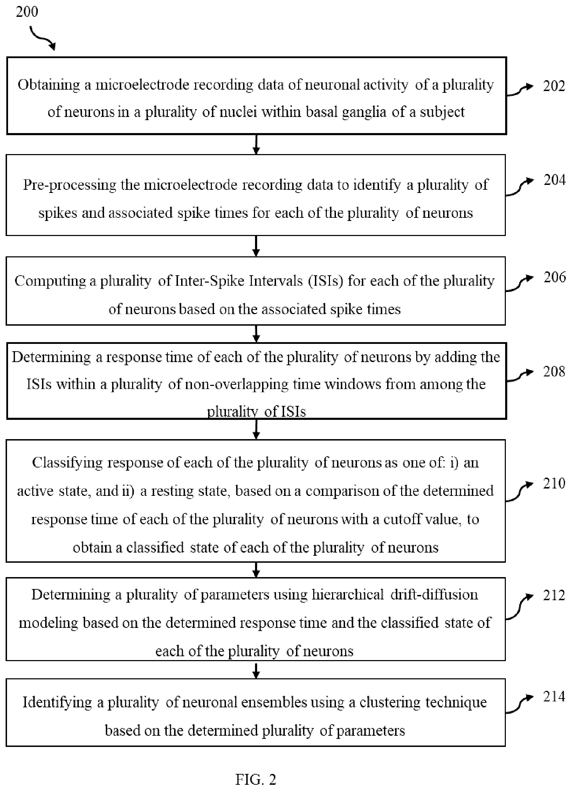

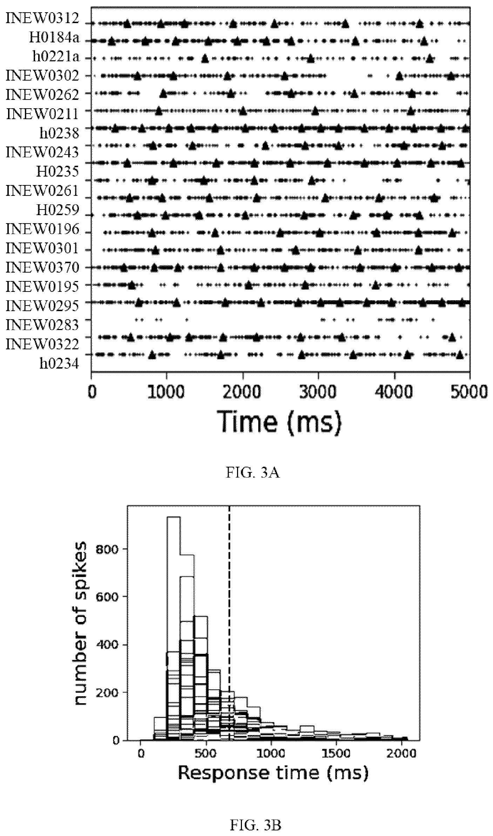

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. The memory 102 may include any computer-readable medium known in the art including, for example, volatile memory, such as Static Random-Access Memory (SRAM) and Dynamic Random-Access Memory (DRAM), and/or non-volatile memory, such as read only memory (ROM), erasable programmable ROM, flash memories, hard disks, optical disks, and magnetic tapes. The database 108 stores information pertaining to inputs fed to the system 100 and/or outputs generated by the system (e.g., at each stage), specific to the methodology described herein. Functions of the components of system 100 are explained in conjunction with flow diagram depicted in FIG. 2, and experimental results illustrated in FIGS. 3A to 6 to identify neuronal ensembles in basal ganglia using hierarchical drift-diffusion modeling.

In an embodiment, the system 100 comprises one or more data storage devices or the memory 102 operatively coupled to the processor(s) 104 and is configured to store instructions for execution of steps of the method 200 depicted in FIG. 2 by the processor(s) or one or more hardware processors 104. The steps of the method of the present disclosure will now be explained with reference to the components or blocks of the system 100 as depicted in FIG. 1, and experimental results illustrated in FIGS. 3A to 6. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

FIG. 2 is a flow diagram illustrating a method 200 to identify neuronal ensembles in basal ganglia using hierarchical drift-diffusion modeling, according to some embodiments of the present disclosure. At step 202, the one or more hardware processors 104 are configured to obtain a Micro-Electrode Recording (MER) data of neuronal activity of a plurality of neurons in a plurality of nuclei within basal ganglia (BG) of a subject. The subject may be a person being monitored for movement-related disorders such as Parkinson Disease (PD), Dystonia etc. which are caused by the plurality of neurons in the BG. As understood by a person skilled in the art, MER involves placement of a fine micro-electrode wire along a planned depth and trajectory to a target location within the BG of the subject. The micro-electrode is advanced using a fine resolution Microdrive until it enters the target location (or target structure) where neuronal activity is recorded. MER records discharge patterns of each of the plurality of neurons that identify and confirm their location(s) within target structures. Characteristic patterns of neuronal activity produced by the neurons have been associated with nuclei such as GPe, GPi and STN as well as other adjacent neural structures such as the thalamus and Substantia Nigra. For example, in PD, characteristic patterns of burst activity are recorded from STN neurons while in Dystonia, characteristic sustained high frequency neuronal discharge is recorded from GP neurons.

Further, at step 204 of the method 200, the one or more hardware processors 104 are configured to pre-process the MER data to identify a plurality of spikes and associated spike times for each of the plurality of neurons. In an embodiment, the MER data is pre-processed by using techniques such as bandpass filtering, spike detection, spike sorting and the like. Bandpass filtering is performed to remove noise from the MER data. Spike detection is generally performed through peak detection algorithms either by using a thresholding approach, or by using techniques like wavelet transforms. Spike sorting is conducted via clustering to identify spike times for specific neurons belonging to particular nuclei. Different pre-processing techniques may be performed in alternate embodiments depending on quality of the MER data.

Further, at step 206 of the method 200, the one or more hardware processors 104 are configured to compute a plurality of Inter-Spike Intervals (ISIs) for each of the plurality of neurons based on the associated spike times. ISIs are computed as difference between each consecutive pair of spike times. This involves subtracting the timestamp of the first spike from the timestamp of the next spike. The ISIs are characterized by equation 1, wherein $j \in \{1,2,...N_B\}$ denotes the plurality of neurons in each of the plurality of nuclei $B$ (for example, $B \in \{GPe, GPi\ and\ STN\}$) within the BG. For example, consider a list of spike times denoted as $[t_1, t_2, t_3, \ldots, t_n]$. The first ISI $\Delta t_1^j$ is the time difference between the second spike time and the first spike time, i.e., $t_2 - t_1$. Similarly, the second ISI $\Delta t_2^j$ is the time difference between the third spike time and the second spike time, i.e., $t_3 - t_2$. This process is continued for all consecutive pairs of spikes. In general, for the i$^{th}$ interval, the ISI $\Delta t_i^j$ is computed as the difference of (i+1)$^{th}$ spike time and i$^{th}$ spike time.

$$\Delta t^j = [\Delta t_1^j, \Delta t_2^j, \ldots] \qquad \ldots\ldots (1)$$

Once the ISIs are computed, at step 208, the one or more hardware processors 104 are configured to determine a response time of each of the plurality of neurons by adding the ISIs within a plurality of non-overlapping time windows from among the plurality of ISIs. The response time $t$ of each of the plurality of neurons denotes time taken by each of the plurality of neurons to reach a predefined spike count threshold $\theta_B$ (for example, $\theta_B$ varies from 20 to 50). The spike count threshold is defined based on required firing rate from a nucleus $B$ in order to pass information, which can be obtained from experimental studies. The response time is calculated according to equation 2, wherein $t_k^j$ is k$^{th}$ response time of neuron $j$ ($j \in \{1,2,...N_B\}$) in a nucleus $B$.

$$t^j = [t_1^j, t_2^j, \ldots], \text{ where } t_k^j = \sum_{i=(k-1)\times\theta_B+1}^{k\times\theta_B} \Delta t_i^j \qquad \ldots (2)$$

During random movement, the activity within the nucleus $B$ undergoes changes. When a neuron reaches the spike count threshold $\theta_B$ more quickly, it tends to exhibit lower response times. This accelerated spiking activity correlates with a higher average drift component of synaptic input to the neuron. Such responses can be characterized as 'active state' responses. Conversely, responses with longer response times are defined as 'resting state' responses. In essence, the response time required for each neuron to reach $\theta_B$ encodes the state of the BG network. Hence, once the response times are determined, at step 210, the one or more hardware processors 104 are configured to classify response of each of the plurality of neurons as one of: i) an active state, and ii) a resting state, based on a comparison of the determined response time of each of the plurality of neurons with a cutoff value, to obtain a classified state of each of the plurality of neurons. The cutoff value $t_{cutoff}$ is determined by at least one of: i) analyzing probability density function of the response time of the plurality of neurons (according to equation 3), and ii) experimental studies of the plurality of nuclei in a disease condition. In equation 3, $PDF(t^j)$ is the probability density function of the response time of neuron $j$ and $t_{cutoff}$ is empirically determined such that 70% of the cumulative density function of the response time lies below the $t_{cutoff}$ value. The cutoff value can also be derived from experimental data or published literature where the correlation between behavioral tasks and neuronal activities has been analyzed simultaneously.

$$t_{cutoff} = 0.7 \times \sum_{j=1}^{N_B} PDF(t^j) \qquad ..... (3)$$

Once the response times and classified state are determined, at step 212 of the method 200, the one or more hardware processors 104 are configured to determine a plurality of parameters using Hierarchical Drift-Diffusion Modeling (HDDM) based on the determined response time and the classified state of each of the plurality of neurons. The plurality of parameters comprise drift rate, threshold, bias, and non-decision time. HDDM is a Python framework for estimating the plurality of parameters using Bayesian inferencing technique. It utilizes observed data i.e. response times $t^j$ to iteratively update beliefs about these parameters, accommodating uncertainty. The classified state of each of the plurality of neurons are used as decision outcomes for associated nucleus. Mathematically, Bayesian inferencing involves specifying prior distributions ($P(\theta)$) for each parameter and computing posterior distribution ($P(\theta|t^j)$) using Bayes' theorem. Markov Chain Monte Carlo (MCMC) sampling technique is employed to approximate the posterior distribution by generating parameter samples from their joint distribution. At each step, the observed response time distribution is compared with the response time distribution given by the HDDM framework according to equation 4.

$$f(t|v, a, z) = \frac{\pi}{a^2} exp(-vaz - \frac{v^2 t}{2}) \times \sum_{l=1}^{\infty} l exp(-\frac{l^2 \pi^2 t}{2a^2}) sin(l\pi z) \quad ..... (4)$$

In equation 4, function $f$ gives probability of response time $t \in t^j$, given threshold $a$, drift rate $v$, and bias b. $l$ is a constant which denotes infinite sum. Non-decision time leads to an offset in response times. Individual response times and decisions are governed by the drift diffusion dynamics described in equations 5 and 6, where $x$ represents distance from either decision boundary at $x = a$ and $x = 0$, and $\eta(t)$ denotes noise.

$$\frac{dx}{dt} = v + \eta(t) \qquad ..... (5)$$

$$Decision = \begin{cases} 1 \ if \ x > a \ (active \ state) \\ 0 \ if \ x < 0 \ (resting \ state) \end{cases} \qquad ..... (6)$$

It was observed that spatially co-located neurons are likely to share similar potentials and input currents, resulting in similar membrane potential evolution. For spatially non-localized neurons, comparable membrane potential evolution can occur when multiple sets of neurons share similar presynaptic inputs and connection weights. If the connection weight between a pair of neurons is high, the post-synaptic current of one neuron is expected to influence the behavior of the other neurons. Consequently, the posterior distribution of drift rate corresponding to each neuron reflects the behavior of the BG network. The drift rate posterior distribution obtained in step 212 is then partitioned into n bins and treated as feature vector of the neuron. This yields an $N_B \times n$ feature matrix for each nucleus $B$. Further, at step 214 of the method 200, the one or more hardware processors are configured to identify a plurality of neuronal ensembles using a clustering technique based on the determined plurality of parameters. The feature matrix is given as input to the clustering technique (such as DBSCAN (Density-Based Spatial Clustering of Applications with Noise)). A distance measure (such as Bhattacharyya distance, Euclidean distance, Manhattan distance, Pearson correlation and the like) is used to quantify the similarity between feature vectors in the clustering technique, where closely associated neurons exhibit lower distances. The minimum number of neurons required per cluster (neuronal ensemble) may be predetermined based on the number of neurons in the obtained MER data in each nucleus B, in either healthy or PD condition. The radius of the clusters are iteratively adjusted until all the plurality of neurons are merged into a single cluster. The clusters formed at each cluster radius are analyzed separately for different nuclei in healthy and PD conditions.

However, as radius of the clusters are increased iteratively, there is a risk of merging uncorrelated neurons into a single large cluster. To address this issue, the clusters are evaluated using a metric such as silhouette score. It assesses clustering quality by measuring how closely each neuron within a cluster resembles its own cluster compared to others.

The silhouette score (*s*) for a specific point within a cluster provides a comparison between the average intra-cluster distance ($d_1$) and the average nearest cluster distance ($d_2$) and is defined according to equation 7. If every neuron within each cluster achieves a silhouette score greater than zero, the corresponding cluster radius is deemed to generate good clusters.

$$s = \frac{(d_1 - d_2)}{max(d_1, d_2)} \qquad \qquad \ldots (7)$$

APPLICATIONS

Deep brain stimulation (DBS) is a surgical therapy used to treat certain aspects of Parkinson's disease (PD). During DBS, doctors usually select a set of particular neurons at specific location in STN. Currently, the selection is based on the symptomatic relief doctor is seeing in the patient, but more the number of neurons being stimulated, more will be side effects. Hence selection of optimal set of neurons is required which can be achieved by identifying neuronal ensembles that are behaving similarly using method 200.

In order to study any disease progression or study neuronal dynamics, researchers need to study a bunch of neurons in animal or human experiments. In such cases, the study can be made simpler by identifying neuronal ensembles and focus the study on the desired neuronal ensemble. Furthermore, during decision making process where BG takes pivotal role, it is observed that a bunch of neurons forms spatiotemporal clusters based on their characteristics. Method 200 can be used to identify such clusters based on hidden properties which govern the neuronal activity. Once the neuronal clusters are identified, then, instead of analysing or stimulating all the neurons, the researchers can pick one cluster and analyse or stimulate them.

EXPERIMENTAL SETUP AND RESULTS

Data description: For the experiments, publicly available ISI data from the work conducted by Adriana et al. ("Basal ganglia neurons in healthy and parkinsonian primates generate recurring sequences of spikes," Journal of Neurophysiology, vol. 129, no. 5, pp. 1010-1020, 2023.) was utilized. The data comprised of electrophysiological recordings from extracellular sources conducted on freely moving rhesus monkeys under both healthy and PD conditions. The recordings encompassed activity from individual neurons in the BG nuclei GPe, GPi and STN, with recorded neuron identities varied between PD and healthy condition. The recording process occurred asynchronously across the different nuclei and all individual neurons. Since the monkey was allowed to freely move throughout the data collection period, it can be assumed that the average dynamics of the BG network remained consistent during this interval while the monkey performed the same motor behaviour repeatedly. As a result, neurons which fall within the same cluster exhibit similar behaviour over a larger time scale, making these ISIs suitable for studying method 200 despite being asynchronous in nature. The neuronal IDs ('N_ID') in the results correspond to those used in Adriana et al.

The input for the HDDM framework was generated using the response time cutoffs between the active and resting state responses as described in equation 3. The response time cutoff chosen must reflect the response time in movement-based tasks observed in real-world experiments. For a given spike count threshold, the fraction of active and resting state responses would depend upon the response time cutoff. The spike count threshold $\theta_B$ would also depend upon the nucleus under consideration, and whether the subject possessed a healthy or PD affected brain. Due to the unavailability of this specific data in movement tasks for rhesus monkeys, $\theta_B = 30$ spikes and $t_{cutoff}$ value corresponding to 70% of responses in active state was used in the experiments. However, the method 200 is robust enough to handle a broad range of $\theta_B$ values.

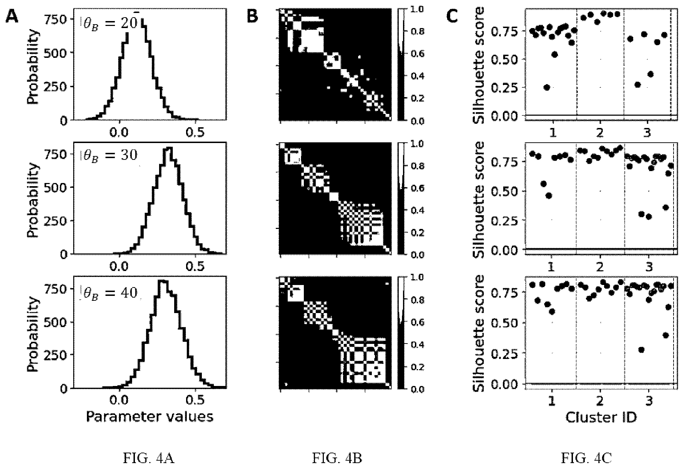

First the spike times derived from ISIs for the GPe in PD condition were considered. FIGS. 3A and 3B, collectively referred to as FIG.3, illustrate spike times and response times of a plurality of neurons, respectively, according to some embodiments of the present disclosure FIG. 3A shows spike times for 20 neurons among $N_B = 40$ neurons. Points marked in triangle markers denote the times at which $\theta_B = 30$ spikes were reached. FIG. 3B shows the response time cutoff $t_{cutoff} = 679ms$ for spike count threshold $\theta_B = 30$ spikes, below which response times were indicative of active state concerning movement. This response time data with defined active and resting state responses was fit using the HDDM framework to obtain the drift rate posterior distribution for each neuron. FIG. 4A illustrates posterior probability distribution of drift rate for a neuron 'H0222' in Globus Pallidus externa (GPe) of the basal ganglia, obtained from Hierarchical Drift-Diffusion Modeling (HDDM), according to some embodiments of the present disclosure. Similarly, drift rates for remaining neurons in the dataset can also be plotted. The posterior distribution of the drift rates for $N_B = 40$ neurons in the GPe in PD condition (nucleus B) was partitioned into n = 10 bins. DBSCAN algorithm was applied to the feature matrix of dimension $40 \times 10$ ($N_B \times n$). The Bhattacharyya distance between each pair of neurons depicted the existence of clusters in GPe. FIG. 4B illustrates Bhattacharyya distance calculated between drift rate posteriors of each pair of neurons, according to some embodiments of the present disclosure. The DBSCAN algorithm was iteratively performed with increasing cluster radius, ensuring that each cluster formed consisted of a minimum of 6 neurons. FIG. 4C shows the formation of cluster in GPe in PD conditions. It is worth noting from FIG. 4B that varying the values of $\theta_B$ also leads to the formation of distinct clusters, indicating the robustness of method 200 across different spike thresholds. In FIG. 4B, each grid point corresponds to a pair of neurons ('N_ID'). Scale or legend of the plot illustrated in FIG. 4B denotes 'Expo'= exp (-Bhattacharyya distance), where 'Expo'= 1 (i.e. distance = 0) signifies a perfect match between two neuron posteriors, resulting in cluster formation. Conversely, higher distances indicate 'Expo' values closer to 0. Presence of clusters of neurons with similar Bhattacharyya distances can be observed in the FIG. 4B out of which 3 are selected as good clusters (neuronal ensembles) based on their Silhouette scores. FIG. 4C illustrates the selected neuronal ensembles with similar drift rate posterior distributions, according to some embodiments of the present disclosure.

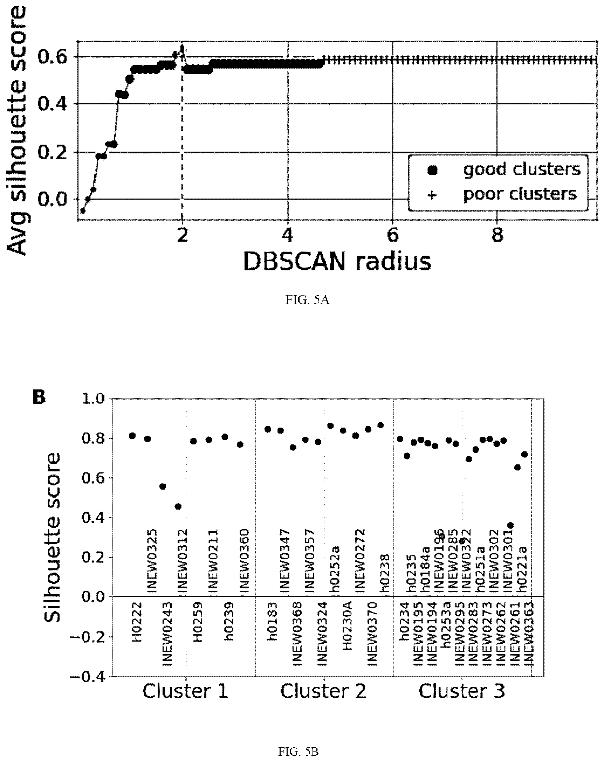

FIG. 5A illustrates evolution of average silhouette score of a plurality of neurons in GPe in Parkinson's Disease (PD) condition with increase in radius of neuronal ensembles for $\theta_B$ = 30 spikes, according to some embodiments of the present disclosure. It can be observed from FIG. 5A that beyond radius = 2, multiple GPe neurons started coalescing into larger clusters in PD condition. Similar neuron clusters were also noted in the GPi and STN in both healthy and PD conditions. The circular points in FIG. 5A correspond to radii which produced 'good clusters' with s > 0 for all clustered neurons. The black dotted line corresponds to the DBSCAN radius with maximum number of good clusters with least fraction of uncorrelated neurons. FIG. 5B illustrates 3 neuronal ensembles (clusters) having least fraction of uncorrelated neurons among the neuronal ensembles illustrated in FIG. 5A, according to some embodiments of the present disclosure. Formation of 3 clusters with DBSCAN radius = 2, fraction of uncorrelated neurons = 0.075 can be seen from FIG. 5B.

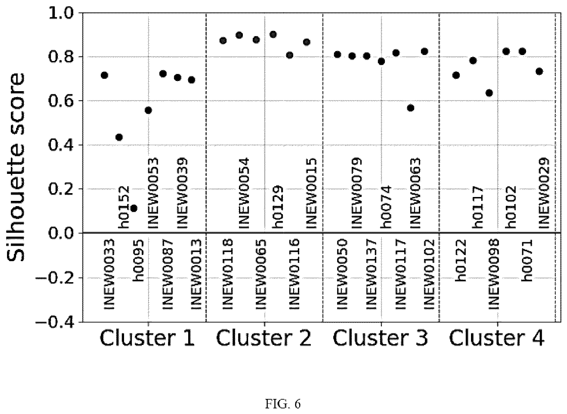

FIG. 6 illustrates a plurality of neuronal ensembles in GPe in healthy condition and their corresponding Silhouette scores, according to some embodiments of the present disclosure. The clustered neurons in both PD and healthy conditions were then analyzed based on two key features, namely LvR and IFR, to determine if they exhibited patterns directly originating from features extracted from ISIs or spike times. These features were extracted using overlapping 3-second windows applied to the ISI data of all neurons. Upon analysis of the overlap in a Kernel Density Estimate (KDE) plot for each individual cluster, a significant degree of similarity (density overlap) between the clusters becomes visible. The consistency of this observation holds true for the neuronal clusters formed in the GPe under healthy conditions as well. This finding implies that solely relying on features derived from recorded observable may make it challenging to observe formation of clusters among neurons. However, upon deeper exploration of the underlying behavior, they successfully coalesce into a cluster. This finding underscores the importance of delving beyond observable data to uncover underlying patterns and relationships within neuronal activity in the BG using the HDDM framework.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, non-volatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method, comprising:

   obtaining (202), via one or more hardware processors, a microelectrode recording data of neuronal activity of a plurality of neurons in a plurality of nuclei within basal ganglia of a subject;
   pre-processing (204), via the one or more hardware processors, the microelectrode recording data to identify a plurality of spikes and associated spike times for each of the plurality of neurons;
   computing (206), via the one or more hardware processors, a plurality of Inter-Spike Intervals (ISIs) for each of the plurality of neurons based on the associated spike times;
   determining (208), via the one or more hardware processors, a response time of each of the plurality of neurons by adding the ISIs within a plurality of non-overlapping time windows from among the plurality of ISIs;
   classifying (210), via the one or more hardware processors, response of each of the plurality of neurons as one of: i) an active state, and ii) a resting state, based on a comparison of the determined response time of each of the plurality of neurons with a cutoff value, to obtain a classified state of each of the plurality of neurons;
   determining (212), via the one or more hardware processors, a plurality of parameters using hierarchical drift-diffusion modeling based on the determined response time and the classified state of each of the plurality of neurons; and
   identifying (214), via the one or more hardware processors, a plurality of neuronal ensembles using a clustering technique based on the determined plurality of parameters.

2. The method as claimed in claim 1, wherein the response time of each of the plurality of neurons denotes time taken by each of the plurality of neurons to reach a predefined spike count threshold.

3. The method as claimed in claim 1, wherein the cutoff value is determined by at least one of: i) analyzing probability density function of the response time of the plurality of neurons, and ii) experimental studies of the plurality of nuclei in a disease condition.

4. The method as claimed in claim 1, wherein the plurality of parameters comprise drift rate, threshold, bias and non-decision time.

5. A system (100), comprising:

   a memory (102) storing instructions;
   one or more Input/Output (I/O) interfaces (106); and
   one or more hardware processors (104) coupled to the memory (102) via the one or more communication interfaces (106), wherein the one or more hardware processors (104) are configured by the instructions to:

      obtain a microelectrode recording data of neuronal activity of a plurality of neurons in a plurality of nuclei within basal ganglia of a subject;
      pre-process the microelectrode recording data to identify a plurality of spikes and associated spike times for each of the plurality of neurons;

compute a plurality of Inter-Spike Intervals (ISIs) for each of the plurality of neurons based on the associated spike times;

determine a response time of each of the plurality of neurons by adding the ISIs within a plurality of non-overlapping time windows from among the plurality of ISIs;

classify response of each of the plurality of neurons as one of: i) an active state, and ii) a resting state, based on a comparison of the determined response time of each of the plurality of neurons with a cutoff value, to obtain a classified state of each of the plurality of neurons;

determine a plurality of parameters using hierarchical drift-diffusion modeling based on the determined response time and the classified state of each of the plurality of neurons; and

identify a plurality of neuronal ensembles using a clustering technique based on the determined plurality of parameters.

6. The system as claimed in claim 5, wherein the response time of each of the plurality of neurons denotes time taken by each of the plurality of neurons to reach a predefined spike count threshold.

7. The system as claimed in claim 5, wherein the cutoff value is determined by at least one of: i) analyzing probability density function of the response time of the plurality of neurons, and ii) experimental studies of the plurality of nuclei in a disease condition.

8. The system as claimed in claim 5, wherein the plurality of parameters comprise drift rate, threshold, bias and non-decision time.

9. One or more non-transitory machine-readable information storage mediums comprising one or more instructions which when executed by one or more hardware processors cause:

obtaining a microelectrode recording data of neuronal activity of a plurality of neurons in a plurality of nuclei within basal ganglia of a subject;

pre-processing the microelectrode recording data to identify a plurality of spikes and associated spike times for each of the plurality of neurons;

computing a plurality of Inter-Spike Intervals (ISIs) for each of the plurality of neurons based on the associated spike times;

determining a response time of each of the plurality of neurons by adding the ISIs within a plurality of non-overlapping time windows from among the plurality of ISIs;

classifying response of each of the plurality of neurons as one of: i) an active state, and ii) a resting state, based on a comparison of the determined response time of each of the plurality of neurons with a cutoff value, to obtain a classified state of each of the plurality of neurons;

determining a plurality of parameters using hierarchical drift-diffusion modeling based on the determined response time and the classified state of each of the plurality of neurons; and

identifying a plurality of neuronal ensembles using a clustering technique based on the determined plurality of parameters.

10. The one or more non-transitory machine readable information storage mediums in claim 9, wherein the response time of each of the plurality of neurons denotes time taken by each of the plurality of neurons to reach a predefined spike count threshold.

11. The one or more non-transitory machine readable information storage mediums in claim 9, wherein the cutoff value is determined by at least one of: i) analyzing probability density function of the response time of the plurality of neurons, and ii) experimental studies of the plurality of nuclei in a disease condition.

12. The one or more non-transitory machine readable information storage mediums in claim 9, wherein the plurality of parameters comprise drift rate, threshold, bias and non-decision time.

FIG. 1

200

Obtaining a microelectrode recording data of neuronal activity of a plurality of neurons in a plurality of nuclei within basal ganglia of a subject — 202

Pre-processing the microelectrode recording data to identify a plurality of spikes and associated spike times for each of the plurality of neurons — 204

Computing a plurality of Inter-Spike Intervals (ISIs) for each of the plurality of neurons based on the associated spike times — 206

Determining a response time of each of the plurality of neurons by adding the ISIs within a plurality of non-overlapping time windows from among the plurality of ISIs — 208

Classifying response of each of the plurality of neurons as one of: i) an active state, and ii) a resting state, based on a comparison of the determined response time of each of the plurality of neurons with a cutoff value, to obtain a classified state of each of the plurality of neurons — 210

Determining a plurality of parameters using hierarchical drift-diffusion modeling based on the determined response time and the classified state of each of the plurality of neurons — 212

Identifying a plurality of neuronal ensembles using a clustering technique based on the determined plurality of parameters — 214

FIG. 2

FIG. 3A

FIG. 3B

FIG. 4A                    FIG. 4B                    FIG. 4C

FIG. 5A

FIG. 5B

FIG. 6

| | Europäisches Patentamt |
|---|---|
| | European Patent Office |
| | Office européen des brevets |

## EUROPEAN SEARCH REPORT

**Application Number**

EP 25 18 3808

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BELOVA ELENA M ET AL: "Oscillations of pause-burst neurons in the STN correlate with the severity of motor signs in Parkinson's disease", EXPERIMENTAL NEUROLOGY, ELSEVIER, AMSTERDAM, NL, vol. 356, 27 June 2022 (2022-06-27), XP087132865, ISSN: 0014-4886, DOI: 10.1016/J.EXPNEUROL.2022.114155 [retrieved on 2022-06-27] * page 2, column 1, paragraph 3 - column 2, paragraph 3; page 3, column 1, paragraph 2 - 3, column 1, paragraph 5; * | 1-12 | INV. A61B5/16 A61B5/00 G06N3/049 G16H50/20 G16H50/50 |
| A | MURASKIN JORDAN ET AL: "A multimodal encoding model applied to imaging decision-related neural cascades in the human brain", NEUROIMAGE, vol. 180, 30 June 2017 (2017-06-30), pages 211-222, XP085459400, ISSN: 1053-8119, DOI: 10.1016/J.NEUROIMAGE.2017.06.059 * abstract; figures 2-3 * | 1-12 | |
| | | | TECHNICAL FIELDS SEARCHED (IPC) |
| | | | A61B G16H G06F G06N |

-/--

The present search report has been drawn up for all claims

| Place of search | Date of completion of the search | Examiner |
|---|---|---|
| The Hague | 6 August 2025 | Chacon Caldera, J |

EPO FORM 1503 03.82 (P04C01)

Europäisches
Patentamt

European
Patent Office

Office européen
des brevets

# EUROPEAN SEARCH REPORT

**Application Number**

EP 25 18 3808

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MYROV VLADISLAV ET AL: "Neural activity clusterization for estimation of firing pattern", JOURNAL OF NEUROSCIENCE METHODS., [Online] vol. 311, 1 January 2019 (2019-01-01), pages 164-169, XP093302384, NL ISSN: 0165-0270, DOI: 10.1016/j.jneumeth.2018.10.017 Retrieved from the Internet: URL:https://pdf.sciencedirectassets.com/27 1055/1-s2.0-S0165027018X00168/1-s2.0-S0165 02701830325X/main.pdf?hash=7a9fab9c7c23bbd e34f22f1981f5416cf3f0493dbc27086a8e23f15db c4b9986&host=68042c943591013ac2b2430a89b27 0f6af2c76d8dfd086a07176afe7c76c2c61&pii=S0 16502701830325X&tid=spdf-bb64151c-ed16-49a 4-9861-e75> [retrieved on 2025-08-04] * abstract; figures 2-3 * ----- | 1-12 | |
| A | YAU Y ET AL: "Neural Correlates of Evidence and Urgency During Human Perceptual Decision-Making in Dynamically Changing Conditions", CEREBRAL CORTEX, [Online] vol. 30, no. 10, 5 June 2020 (2020-06-05), pages 5471-5483, XP093302057, GB ISSN: 1047-3211, DOI: 10.1093/cercor/bhaa129 Retrieved from the Internet: URL:http://academic.oup.com/cercor/article -pdf/30/10/5471/33713654/bhaa129.pdf> [retrieved on 2025-08-04] * abstract; figures 1,4,5 * -----  -/-- | 1-12 | TECHNICAL FIELDS SEARCHED (IPC) |

The present search report has been drawn up for all claims

| Place of search | Date of completion of the search | Examiner |
|---|---|---|
| The Hague | 6 August 2025 | Chacon Caldera, J |

EPO FORM 1503 03.82 (P04C01)

**page 2 of 3**

| | Europäisches Patentamt European Patent Office Office européen des brevets | **EUROPEAN SEARCH REPORT** | Application Number EP 25 18 3808 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MULDER M J ET AL: "Perceptual decision neurosciences - A model-based re", NEUROSCIENCE, NEW YORK, NY, US, vol. 277, 29 July 2014 (2014-07-29), pages 872-884, XP029054552, ISSN: 0306-4522, DOI: 10.1016/J.NEUROSCIENCE.2014.07.031 * abstract; figures 1,2,7 * ----- | 1-12 | |
| A | FRANK MICHAEL J. ET AL: "fMRI and EEG Predictors of Dynamic Decision Parameters during Human Reinforcement Learning", THE JOURNAL OF NEUROSCIENCE, [Online] vol. 35, no. 2, 14 January 2015 (2015-01-14), pages 485-494, XP093302055, US ISSN: 0270-6474, DOI: 10.1523/JNEUROSCI.2036-14.2015 Retrieved from the Internet: URL:https://www.jneurosci.org/content/jneuro/35/2/485.full.pdf> [retrieved on 2025-08-04] * abstract; figures 4,5 * ----- | 1-12 | |
| X,P | PARI BHUVANAMBIGA ET AL: "Unraveling Neuronal Cluster Dynamics in Basal Ganglia Using Hierarchical Drift-Diffusion Modeling", 2024 32ND EUROPEAN SIGNAL PROCESSING CONFERENCE (EUSIPCO), EUROPEAN ASSOCIATION FOR SIGNAL PROCESSING - EURASIP, 26 August 2024 (2024-08-26), pages 1481-1485, XP034730188, DOI: 10.23919/EUSIPCO63174.2024.10714987 [retrieved on 2024-10-23] * the whole document * ----- | 1-12 | TECHNICAL FIELDS SEARCHED (IPC) |

The present search report has been drawn up for all claims

| Place of search | Date of completion of the search | Examiner |
|---|---|---|
| The Hague | 6 August 2025 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421047653

**Non-patent literature cited in the description**

- **ADRIANA et al.** Basal ganglia neurons in healthy and parkinsonian primates generate recurring sequences of spikes. *Journal of Neurophysiology*, 2023, vol. 129 (5), 1010-1020